(19) Europäisches Patentamt
European Patent Office
Office européen des brevets

(11) Veröffentlichungsnummer: **0 090 231**
**B1**

(12) **EUROPÄISCHE PATENTSCHRIFT**

(45) Veröffentlichungstag der Patentschrift: **13.11.85**

(51) Int. Cl.⁴: **C 07 D 307/58**

(21) Anmeldenummer: **83102450.0**

(22) Anmeldetag: **12.03.83**

(54) **Verfahren zur Herstellung von 3-Alkyl-2,5-dihydrofuran-2-onen.**

(30) Priorität: **24.03.82 DE 3210705**

(43) Veröffentlichungstag der Anmeldung: **05.10.83 Patentblatt 83/40**

(45) Bekanntmachung des Hinweises auf die Patenterteilung: **13.11.85 Patentblatt 85/46**

(84) Benannte Vertragsstaaten: **CH DE FR GB LI**

(56) Entgegenhaltungen:
**EP-A-0 018 162**
**DE-A-2 461 525**

(73) Patentinhaber: **BASF Aktiengesellschaft, Carl-Bosch-Strasse 38, D-6700 Ludwigshafen (DE)**

(72) Erfinder: **Fischer, Rolf, Dr., Bergstrasse 98, D-6900 Heidelberg (DE)**
Erfinder: **Weitz, Hans-Martin, Dr., Auf dem Koeppel 40, D-6702 Bad Duerkheim (DE)**

**EP 0 090 231 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

**Beschreibung**

Die Erfindung betrifft ein Verfahren zur Herstellung von 3-Alkyl-2,5-dihydrofuran-2-onen durch Cyclisierung von Butenderivaten in Gegenwart von sauer wirkenden Verbindungen.

Es sind schon mehrere Verfahren zur Herstellung von 3-Methyl-2,5-dihydrofuran-2-on bekannt. Als Ausgangsverbindungen verwendet man dabei z. B. 1-Hydroxy-3-oxobutan (Journ. Amer. Chem. Soc. 68, 2332 (1946)), 2-Methyl-3-butencarbonsäure (Bull. Soc. Chim. France 1968, 4067), 3-Methylbutyrolacton (Chem. Pharm. Bull. 1973, 461) oder 2-Butin-1-ol (Tetrahedron Letters 1979, 133). Alle diese Synthesen verlaufen jedoch entweder über zahlreiche Stufen oder gehen von nicht einfach zugänglichen Ausgangsverbindungen aus.

Es wurde nun gefunden, daß man 3-Alkyl-2,5-dihydrofuran-2-one der Formel

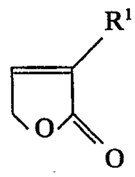

(I)

in der $R^1$ einen Alkylrest mit 1 bis 15 Kohlenstoffatomen bedeutet, besonders vorteilhaft dadurch herstellen kann, daß man Butenderivate der Formeln

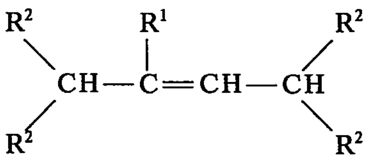

(II)

oder

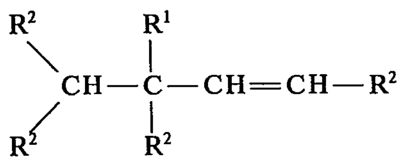

(III)

in denen $R^1$ einen Alkylrest mit 1 bis 15 Kohlenstoffatomen, die Reste $R^2$ jeweils einen Acyloxyrest der Formel

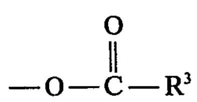

in der $R^3$ für einen Alkylrest mit 1 bis 5 Kohlenstoffatomen oder einen Aryl- oder Cycloalkylrest mit 5 bis 7 Kohlenstoffatomen steht, oder einen Alkoxyrest mit 1 bis 5 Kohlenstoffatomen bedeuten, oder in denen zwei Reste $R^2$ am gleichen Kohlenstoffatom zusammen ein Sauerstoffatom oder einen

$-O-(CH_2)_x-O$-Rest

mit $x = 2$ oder 3 bedeuten, mit sauer wirkenden Mitteln behandelt.

Dieses neue Verfahren ermöglicht eine vorteilhafte Synthese der Alkyldihydrofuran-2-one, weil als Ausgangsstoffe nicht nur die nach dem in der DE-OS 3 125 891 beschriebenen Verfahren durch Umsetzung von 2-Alkyl-1,4-diacyloxy-1,3-butadienen mit Epoxidierungsmitteln erhältlichen 2-Alkyl-4,4-diacyloxy-2-butenale, sondern auch die dabei als Nebenprodukte anfallenden Gemische aus 2-Alkyl-2,4-diacyloxy-3-butenalen, 3-Alkyl-4,4-diacyloxy-2-butenalen, 2-Alkyl-1,1,4,4-tetraacyloxy-2-butene und 2-Alkyl-2-buten-1,4-dialen, die noch geringe Mengen an 2-Alkyl-4,4-diacyloxy-2-butenalen enthalten können, geeignet sind. Daß eine vorherige Trennung dieser Gemische nicht erforderlich ist, ist ebenso überraschend wie die vorteilhafte Tatsache, daß im Reaktionsprodukt keine 4-Alkyl-2,5-dihydrofuran-2-one gefunden werden.

Das erfindungsgemäße Verfahren läßt sich am Beispiel der Umsetzung von 2-Methyl-2-buten-1,4-dial in Gegenwart von wäßriger Essigsäure zu 3-Methyl-2,5-dihydrofuran-2-on formal durch das folgende Formelschema wiedergeben:

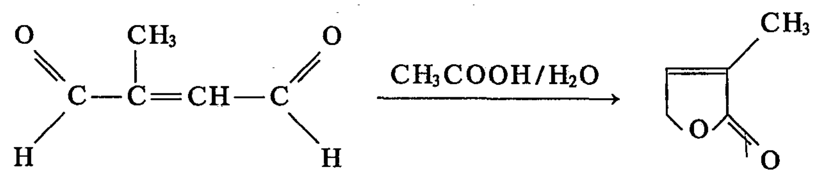


Die Ausgangsverbindungen der Formel II und III enthalten als Alkylreste $R^1$, z. B. Alkylgruppen mit 1 bis 15, vorzugsweise 1 bis 5 Kohlenstoffatomen. Die Alkylreste $R^3$ haben 1 bis 5, vorzugsweise 1 bis 3 Kohlenstoffatome. Alkylreste der genannten Art sind z. B. Methyl-, Ethyl-, n-Propyl-, i-Propyl-, n-Butyl-, i-Butyl- oder tert.-Butylreste. Als Cycloalkyl- oder Arylreste kommen z. B. Cyclohexyl- und Phenylreste in Betracht. Alkoxyreste sind z. B. Methoxy- oder Ethoxy-Reste. Bevorzugte Ausgangsstoffe sind z. B. 2-Methyl-4,4-diacetoxy-2-butenal, 2-Methyl-1,1,4,4-tetraacetoxy-2-buten, 2-Methyl-2-buten-1,4-dial, 3-Methyl-4,4-diacetoxy-2-butenal und 2-Methyl-2,4-diacetoxy-3-butenal, die man in Form der reinen Verbindungen, vor allem aber auch als Gemisch einsetzen kann. Diese Ausgangsstoffe sind z. B. durch Umsetzung der entsprechenden 2-Alkyl-1,4-diacyloxy-1,3-butadiene mit Carbonsäuren und Sauerstoff oder Sauerstoff abgebenden Verbindungen (DE-OS 3 125 891) herstellbar.

Die Umsetzung der Butenderivate zur Herstellung der 3-Alkyl-2,5-dihydrofuran-2-one führt man im allgemeinen bei Temperaturen von 0 bis 200°C, vorzugsweise bei 80 bis 120°C, drucklos oder unter Druck, diskontinuierlich oder kontinuierlich durch.

Als sauer wirkende Mittel verwendet man z. B. aliphatische Carbonsäuren, wie Ameisensäure, Essigsäure, Propionsäuren oder Valeriansäure, Sulfonsäuren, wie p-Toluolsulfonsäuren, Mineralsäuren, wie Salzsäure, Schwefelsäure oder Bromwasserstoffsäure. Weiterhin lassen sich auch Kationenaustauscher in ihrer Säureform verwenden, z. B. Austauscher, die aus Styrol und Divinylbenzol aufgebaut sind und Sulfonsäuregruppen enthalten, oder anorganische Kationenaustauscher, wie Zeolithe, Phenol- oder Polystyrolsulfonsäureharze, Styrolphosphonsäureharze, Styrolphosphinsäureharze, oder entsprechende saure Harze enthaltende Austauscher, z. B. bifunktionelle Kondensationsharze.

Kationenaustauscher der genannten Art sind z. B. die im Handel unter den Bezeichnungen ®Lewatit S 100, ®Amberlit IR-120, ®Lewasorb, ®Dowex 50 WX 8, ®Amberlyst 15 erhältlichen Produkte. Als sauer wirkende Verbindungen kann man auch Lewissäuren, wie Bortrifluorid, Zinkchlorid, Eisen(III)chlorid, Aluminiumchlorid oder Zinn(II)chlorid verwenden.

Die Carbonsäuren der genannten Art werden z. B. in Mengen von 0,1 bis 50 Molen pro Mol der Ausgangsstoffe II und III angewendet. Bei Verwendung der Sulfonsäuren oder der starken Mineralsäuren und Lewissäuren genügen katalytische Mengen, wie 0,002 bis 0,25 Äquivalente der Säuren pro Mol der Verbindung II oder III. Die Menge der Kationenaustauscher hängt von der Selektivität bzw. der Zahl der austauschaktiven Gruppen der verwendeten Austauscher bei der Reaktionstemperatur ab. Im allgemeinen verwendet man 1 bis 40 Gewichtsprozent, vorzugsweise 5 bis 25 Gewichtsprozent Austauscher, bezogen auf die Ausgangsstoffe II und III.

Man kann das erfindungsgemäße Verfahren auch in einem unter den Reaktionsbedingungen inerten Lösungsmittel durchführen. Diese Arbeitsweise empfiehlt sich besonders dann, wenn man als sauer wirkende Verbindung keine Carbonsäure verwendet, die gleichermaßen als Lösungsmittel wirkt.

Unter den Reaktionsbedingungen inerte Lösungsmittel sind z. B. Wasser, Alkohole, wie Ethanol, Methanol, n-Butanol, Isobutanol, tert.-Butanol, Glykol, Glycerin, sek.-Butanol oder Cyclohexanol, chlorierte Kohlenwasserstoffe, wie Chloroform, Methylenchlorid oder Tetrachlorkohlenstoff, Ether, wie Diethylether, Tetrahydrofuran oder Dioxan sowie Gemische derartiger Lösungsmittel.

Bei einer bevorzugten Ausführungsform des Verfahrens führt man die Umsetzung in einer Carbonsäure, wie Ameisensäure, Essigsäure, Propionsäure oder Valeriansäure durch, wobei man geringe Mengen Wasser zusetzt. Die Wassermenge beträgt z. B. 0,001 Mol bis 1 Mol pro Mol Carbonsäure, insbesondere 0,1 bis 0,5 Mole Wasser pro Mol Carbonsäure. Besonders vorteilhaft an dieser Arbeitsweise ist, daß die Carbonsäuren und das Wasser bei der Aufarbeitung destillativ, also ohne Neutralisation mit Basen, entfernt werden können.

Die Umsetzung der Butenderivate nimmt man z. B. so vor, daß man ein Gemisch aus dem Ausgangsstoff II und/oder III, einem Lösungsmittel, den saurem Katalysator und gegebenenfalls Wasser, 0,5 bis 50 Stunden bei der Reaktionstemperatur hält. Dann werden, gegebenenfalls nach Neutralisation des sauren Katalysators, das Lösungsmittel, gegebenenfalls Wasser und niedrigsiedende Nebenprodukte abgezogen. Die 3-Alkyl-2,5-dihydrofuran-2-one werden schließlich durch Destillation gewonnen.

Die nach dem Verfahren der Erfindung erhältlichen 3-Alkyl-2,5-dihydrofuran-2-one gehören zur Stoffklasse der Butenolide, die als Bestandteil einiger Naturstoffe, wie der Cardenolide, der Ascorbinsäure und der Tetronsäuren Bedeutung besitzen. 3-Alkyl-2,5-dihydrofuran-2-one stellen daher interessante Zwischenprodukte dar. 3-Methyl-2,5-dihydrofuran-2-on läßt sich z. B. zu 3-Methyl-5-brom-2,5-dihydrofuran-2-on bromieren, das z. B. zum Aufbau biologisch aktiver Verbindungen verwendbar ist (Aust. J. Chem. 1491, 1974; J. Chem. Soc., Perkin Trans. 1, 410 (1976); DE-PS 2 225 149).

## Beispiel 1

100 g 2-Methyl-4,4-diacetoxy-2-butenal wurden in 1045 g Essigsäure, die 4,3 Gew.-% Wasser enthielt, gelöst. Die Lösung wurde 24 Stunden unter Rückfluß erhitzt. Nach dem Abziehen von Essigsäure und Wasser am Rotationsverdampfer wurden durch fraktionierte Destillation 22,3 g 3-Methyl-2,5-dihydrofuran-2-on vom Siedepunkt 46 bis 48° C/0,3 mbar (45%, bezogen auf eingesetztes 2-Methyl-4,4-diacetoxy-2-butenal) erhalten.

## Beispiel 2

Ein Gemisch aus 200 g 2-Methyl-4,4-diacetoxy-2-butenal, 1500 g Essigsäure und 1,9 g p-Toluolsulfonsäure wurde 4,5 Stunden bei 100° C gerührt, anschließend am Rotationsverdampfer eingedampft und fraktioniert destilliert. Es wurden 55,1 g 3-Methyl-2,5-dihydrofuran-2-on vom Siedepunkt 57° C/1 mbar (56%, bezogen auf eingsetztes 2-Methyl-4,4-diacetoxy-2-butenal) erhalten.

## Beispiel 3

Ein Gemisch aus 100 g 2-Methyl-4,4-diacetoxy-2-butenal, 1000 g Essigsäure und 0,98 g konzentrierter Schwefelsäure wurde unter Stickstoff 50 Minuten bei 100° C gerührt. Das Reaktionsgemisch wurde nach Zugabe von 1,7 g festem Natriumhydrogencarbonat am Rotationsverdampfer eingeengt. Durch fraktionierte Destillation des Rückstands wurden 28,1 g 3-Methyl-2,5-dihydrofuran-2-on vom Siedepunkt 45 bis 52° C/0,8 mbar (57%, bezogen auf eingesetztes 2-Methyl-4,4-diacetoxy-2-butenal) erhalten.

## Beispiel 4

7,5 g 2-Methyl-1,1,4,4-tetraacetoxy-2-buten (Schmelzpunkt 96,5 bis 97,5° C) wurden in 104,5 g Essigsäure, die 4,3 Gew.-% Wasser enthielt, gelöst. Die Lösung wurde 20 Stunden unter Rückfluß erhitzt. Nach der in Beispiel 1 beschriebenen Aufarbeitung wurden 1,5 g 3-Methyl-2,5-dihydrofuran-2-on vom Siedepunkt 40 bis 52° C/0,7 mbar $n_D^{20} = 1{,}4650$ (61%, bezogen auf eingesetztes 2-Methyl-1,1,4,4-tetraacetoxy-2-buten) erhalten.

## Beispiel 5

2,45 g 2-Methyl-2-buten-1,4-dial wurden in 104,5 g Essigsäure, die 4,3 Gew.-% Wasser enthielt, 22 Stunden unter Rückfluß erhitzt. Nach der in Beispiel 1 beschriebenen Aufarbeitung wurden 1,3 g 3-Methyl-2,5-dihydrofuran-2-on vom Siedepunkt 40 bis 48° C/0,3 mbar (53%, bezogen auf eingesetztes 2-Methyl-2-buten-1,4-dial) erhalten.

## Beispiel 6

Ein Gemisch aus 2,9 g 2-Methyl-2,4-diacetoxy-3-butenal und 1,5 g 2-Methyl-4,4-diacetoxy-2-butenal wurde in 50 g Essigsäure gelöst, die 0,025 g konzentrierte Schwefelsäure enthielt. Das Gemisch wurde 20 Minuten bei 100° C unter Stickstoff gerührt. Die Schwefelsäure wurde dann mit festem Natriumbicarbonat neutralisiert und die Essigsäure am Rotationsverdampfer abgezogen. Durch Kugelrohrdestillation (70 bis 150° C/1 mbar) des Rückstands wurden 1,1 g 3-Methyl-2,5-dihydrofuran-2-on (51%, bezogen auf eingesetzte Methyldiacetoxybutenale) erhalten.

## Beispiel 7

Ein Gemisch aus 2,4 g 3-Methyl-4,4-diacetoxy-2-butenal, 0,7 g 2-Methyl-4,4-diacetoxy-2-butenal und 1,2 g 2-Methyl-2,4-diacetoxy-3-butenal wurde in 54,5 g Essigsäure, die 8,25 Gew.-% Wasser enthielt, 32 Stunden auf 100° C erhitzt. Nach der in Beispiel 1 beschriebenen Aufarbeitung wurden 0,9 g 3-Methyl-2,5-dihydrofuran-2-on (43%, bezogen auf eingesetzte Methyldiacetoxybutenale) erhalten.

Beispiel 8

Ein Gemisch aus 100 g 2-Methyl-4,4-diacetoxy-2-butenal, 0,95 g p-Toluolsulfonsäure, 90 g Wasser und 1000 g Dioxan wurde 32 Stunden unter Rückfluß erhitzt. Nach dem Abziehen von Dioxan und Wasser wurde fraktioniert destilliert. Es wurden 17 g 3-Methyl-2,5-dihydrofuran-2-on vom Siedepunkt 62 bis 65° C/1,7 mbar, $n_D^{20} = 1{,}4652$ (33%, bezogen auf eingesetztes 2-Methyl-4,4-diacetoxy-2-butenal) erhalten.

Beispiel 9

25 g des sauren Ionenaustauschers ®Amberlite IR-120 wurde in einer Lösung von 100 g 2-Methyl-4,4-diacetoxy-2-butenal in 500 g Eisessig suspendiert. Die Suspension wurde 8 Stunden bei einer Temperatur von 100° C gerührt. Nach dem Abfiltrieren des Ionenaustauschers und Abziehen der Essigsäure wurden durch fraktionierte Destillation 24,5 g 3-Methyl-2,5-dihydrofuran-2-on (50%, bezogen auf eingesetztes 2-Methyl-4,4-diacetoxy-2-butenal) erhalten.

**Patentansprüche**

1. Verfahren zur Herstellung von 3-Alkyl-2,5-dihydrofuran-2-onen der Formel

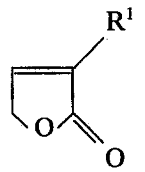

(I)

in der $R^1$ einen Alkylrest mit 1 bis 15 Kohlenstoffatomen bedeutet, dadurch gekennzeichnet, daß man Butenderivate der Formeln

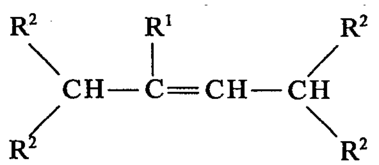

(II)

oder

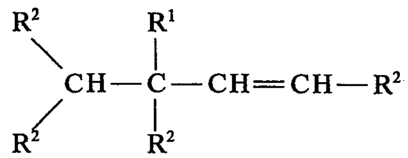

(III)

in denen $R^1$ einen Alkylrest mit 1 bis 15 Kohlenstoffatomen, die Reste $R^2$ jeweils einen Acyloxyrest der Formel

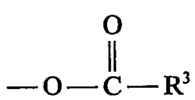

in der $R^3$ für einen Alkylrest mit 1 bis 15 Kohlenstoffatomen oder einen Aryl- oder Cycloalkylrest mit 5 bis 7 Kohlenstoffatomen steht, oder einen Alkoxyrest mit 1 bis 5 Kohlenstoffatomen bedeuten, oder in denen zwei Reste $R^2$ am gleichen Kohlenstoffatom zusammen ein Sauerstoffatom oder einen

$-O-(CH_2)_x-O$-Rest

mit $x = 2$ oder 3 bedeuten, mit sauer wirkenden Mitteln behandelt.

2. Verfahren nach Anspruch 1, dadurch gekennzeichnet, daß man als sauer wirkende Mittel Mineralsäuren, saure Ionenaustauscher oder Wasser enthaltende aliphatische Carbonsäuren verwendet.

**Claims**

1. A process for the preparation of a 3-alkyl-2,5-dihydrofuran-2-one of the formula

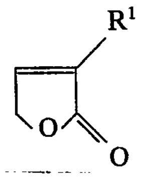

(I)

where $R^1$ is alkyl of 1 to 15 carbon atoms, wherein a butene derivative of the formula

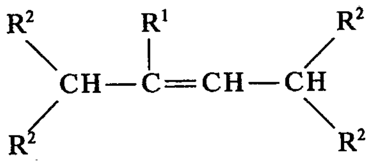

(II)

or

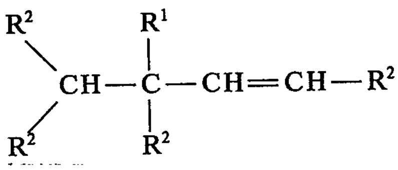

(III)

where $R^1$ is alkyl of 1 to 15 carbon atoms, and the $R^2$'s each denote an acyloxy radical of the formula

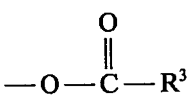

where $R^3$ is alkyl of 1 to 5 carbon atoms, aryl or cycloalkyl of 5 to 7 carbon atoms, or alkoxy of 1 to 5 carbon atoms,
or where two $R^2$'s on the same carbon atom together denote oxygen or

$-O-(CH_2)_x-O-$

where x is 2 or 3, is treated with an acidic agent.

2. A process as claimed in claim 1, wherein the acidic agent used is a mineral acid, an acid ion exchanger or a water-containing aliphatic carboxylic acid.

**Revendications**

1. Procédé pour la préparation de 3-alkyl-2,5-dihydrofuran-2-ones de formule

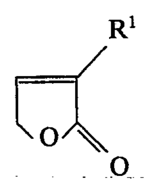

(I)

dans laquelle $R^1$ représente un radical alkyle à 1—15 atomes de carbone, caractérisé en ce qu'on traite par des agents à action acide des dérivés du butène de formules

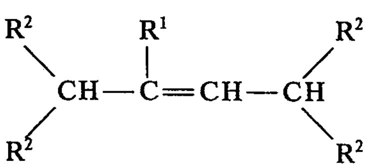

(II)

ou

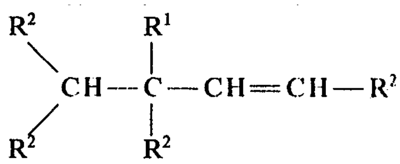

(III)

dans lesquelles $R^1$ représente un radical alkyle à 1—15 atomes de carbone, les symboles $R^2$ représentent chacun un radical acyloxy de formule

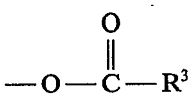

dans laquelle $R^3$ est mis pour un radical alkyle à 1—5 atomes de carbone ou pour un radical aryle ou cycloalkyle à 5—7 atomes de carbone, ou un radical alcoxy à 1—5 atomes de carbone, ou dans lesquelles deux radicaux $R^2$ sur le même atome de carbone représentent un atome d'oxygène ou un radical

$-O-(CH_2)_x-O$-Rest

avec $x=2$ ou 3.

2. Procédé selon la revendication 1, caractérisé en ce qu'on utilise, en tant qu'agents à action acide, des acides minéraux, des échangeurs d'ions acides ou des acides carboxyliques aliphatiques contenant de l'eau.